# EUROPEAN PATENT APPLICATION

(11) **EP 2 338 453 A1**
(43) Date of publication of application: **29.06.2011**
(21) Application number: 09180689.3
(22) Date of filing: 23.12.2009
(51) Int. Cl.: A61F 13/56, A61F 13/58

(54) **Hygiene Article**

(71) Applicant: 3M Innovative Properties Company, Saint Paul, MN 55133-3427 (US)
(72) Inventor: Petersen, Johann, 41516 Grevenbroich (DE); Hauschildt, Volker, 40723 Hilden (DE)
(74) Representative: Wilhelm, Stefan

(57) **Abstract**

A hygiene article, particularly a sanitary napkin or an adult incontinence pad is disclosed. The hygiene article comprises an absorbent core having an elongate shape, being sandwiched between a back sheet and a front sheet. The back sheet and the front sheet extend beyond the absorbent core and are sealed together to form a sealing edge portion. A region of material adapted to form a first fastening means is arranged on the back sheet. A region of material adapted to form a second fastening means, substantially co-extensive with, and adapted to form a protective cover for, the first fastening means is also provided. Preferably at least the second fastening means is provided on the sealing edge. The fastening means may be formed from the same materials, for example, a hermaphroditic fastener, or from different materials for example, a mechanical fastening means and an adhesive fastening means.

## Description

The present invention relates to a hygiene article, in particular, a hygiene article having an elongate absorbent core sandwiched between a back sheet and a front sheet, suitable for use as sanitary napkin or an adult incontinence pad.

Hygiene articles, particularly disposable hygiene articles have a variety of uses, including infant diapers (sometimes also known as nappies), feminine hygiene articles (such as sanitary towels or napkins and panty liners), and adult incontinence wear (for example, incontinence pads and disposable undergarments). Each of these articles is designed to absorb and/or retain liquids and other bodily exudates and has in common the need for the article to be fixed in position relative to the body of a wearer. This may be done by adhering the article directly to itself such that it surrounds a wearer (in the case of infant diapers and disposable undergarments) or to an item of clothing (in the case of feminine hygiene articles and adult incontinence pads), or directly to the skin of a user (in the case of feminine hygiene articles and incontinence pads). One of the most popular fixation mechanisms is to adhere the hygiene article to an item of clothing, for example, an undergarment. In general, there are two main ways in which this may be achieved (a) using adhesive fastening means or (b) using mechanical fastening means. Using each of these methods alone has both advantages and disadvantages, but occasionally both methods may be used on a single article. This may be to enhance the adhesive properties of using a single fastening means, for example, to provide additional adhesion by providing an adhesive fastening means on the main body of a hygiene article and mechanical fastening means on a wing, or to provide additional functionality, for example, using a mechanical fastening means to hold a diaper in position and an adhesive fastening means to hold the diaper in a folded position for disposal.

One example of the former dual-use is disclosed in US2003195488, which describes a sanitary napkin having an adhesive strip placed centrally on the rear of the napkin and along its length, and additional wings provided with a hook and loop mechanical fastening system. The sanitary napkin is provided with elasticated and raised side edges that prevent the leakage of fluids and other bodily exudates. In use, the adhesive strip is placed in contact with a wearers' undergarment, holding the sanitary napkin in position. The wings are fastened underneath the crotch portion of the undergarment, with the flaps fastened to each other, pulling the elasticated raised sides downwards and preventing them from pulling upwards and onto the upper absorbent surface of the sanitary napkin. To prevent the adhesive fastening means from damage or contamination in use, a release liner is provided, which is removed by a user just prior to use. The hook and loop fastening mechanism provided on the wings is fastened together until the sanitary napkin is used, to prevent any damage or contamination to either the hook or the loop patch.

An example of the latter dual use is disclosed in US 5,611,789, which is concerned with using an adhesive fastening means provided on a tape tab to hold a used diaper in position when wrapped in on itself for disposal. In use, the diaper is held in position by mechanical fastening means comprising a hook patch positioned on a tab that engages with a loop patch provided as a landing surface on the main body of the diaper. The adhesive tape tab is positioned partially covering the hook patch, enabling the hook patch to be used without movement or removal of the adhesive tape tab, such that the adhesive fastening means is fully protected until it is required for use.

In each of these examples the adhesive fastening means is fully covered by a release liner, whether it is provided as a separate item or is formed by a portion of the mechanical fastening means. However, protection of the mechanical fastening means is not envisaged beyond keeping the hook and loop patch in contact with each other in a fastened position when the article is not in use, and releasing the hook patch from the loop patch at the point that the article is used. This is less than ideal, firstly, if the two types of fastening mechanism are to be used together to provide fixation for the hygiene article, a user must disengage and release both before use, as in US2003195488, or risk that the loop patch is damaged by removal of the hook patch before use, as in both US2003195488 and US 5,611, 789. In addition, neither document considers the situation where no loop patch or specific landing area for the hook patch is provided, for example, where a mechanical fastening means is used to engage directly with the undergarment of a wearer of the hygiene article.

It would therefore be desirable if a method of providing protection for each fastening means without the need to provide additional materials or special areas to which the fastening means can be fastened, which is simple and easy for a user to employ, could be found.

The present invention aims to address the above problems by providing a hygiene article, comprising an absorbent core having an elongate shape sandwiched between a back sheet and a front sheet, the back sheet and the front sheet extending beyond the absorbent core and being sealed together to form a sealing edge portion; a region of material adapted to form a first fastening means arranged on the back sheet; and a region of material adapted to form a second fastening means, substantially co-extensive with, and adapted to form a protective cover for, the first fastening means.

By using a second region of material adapted to form a fastening means to form a protective cover for a first region of material adapted to form a fastening means, there is no need to use a separate release liner. By ensuring that the region of material adapted to form a second fastening means is co-extensive with the region of material adapted to form a first fastening means, that is each region of material adapted to form a second fastening means covers substantially the whole of the region of material adapted to form a first fastening means to which it corresponds, no damage occurs to either fastening means during storage. If the region of material adapted to form a first fastening means is an adhesive fastening means and the second region of material adapted to form a second fastening means is a mechanical fastening means, for example, then there is also no need to supply a separate loop portion to protect the mechanical fastening means, or a separate release liner to protect an adhesive fastening means, and so it can be used on any suitable surface, such as an undergarment, to ensure that the hygiene article is fully secured in use. By adapting materials such as mechanical fasteners, adhesives, hermaphroditic fasteners (where two regions of the same material are able to mesh together to form a fastening means) or releasable adhesives (where two regions of adhesive once placed in contact can be peeled apart easily, thus forming their own release liners) to form a fastening means and a release surface, greater flexibility in both hygiene article design and component materials choices is achieved.

The region of material adapted to form a first fastening means is arranged on the back sheet in the region of the sealing edge. By placing this fastening means close to the sealing edge, when the hygiene article is worn, its edge regions are less likely to curl and/or bunch up, making it more comfortable for a wearer. Preferably though, the region of material adapted to form a first fastening means is arranged on the back sheet on the sealing edge.

Preferably, the region of material adapted to form a second fastening means is arranged on the back sheet on the sealing edge. This is advantageous as the sealing edge may be folded over onto itself easily, as there is no bulky region from the absorbent core.

Preferably, the hygiene article further comprises a first wing having a at least one region of material adapted to form a second fastening means located thereon and a second wing having at least one region of material adapted to form a second fastening means located thereon. The wing is intended to fold around the edge of an undergarment when the hygiene article is worn in use, both securing the hygiene article and preventing leakage of fluids and bodily exudates. The wings are conventionally rectangular lying with the long side of the rectangle along the sealing edge, although many other shapes are suitable, including trapezoidal and semi-circular.

The hygiene article preferably has an elongated shape comprising first and second opposing longitudinal sides (that is, the long sides of a substantially rectangular elongate shape) and first and second opposing transverse sides (that is, the short sides of a substantially rectangular elongate shape), and may have a region of material adapted to form a first fastening means provided on the first longitudinal side and a second region of material adapted to form a first fastening means provided on the second longitudinal side.

The front sheet and/or the back sheet may extend beyond the sealing edge to form the wings. Alternatively, the wings may be discrete and bonded to the hygiene article at the sealing edge.

Preferably, the hygiene article has an elongated shape comprising first and second opposing longitudinal sides (that is, the long sides of a substantially rectangular elongate shape) and first and second opposing transverse sides (that is, the short sides of a substantially rectangular elongate shape), and may have a first region of material adapted to form a first fastening means provided on the first transverse side and a second region of material adapted to form a first fastening means provided on the second transverse side. These fastening means may be provided symmetrically about an axis disposed transversely along the centre of the hygiene article or otherwise.

In this situation, preferably a region of material forming a second fastening means is provided adjacent to each region of material adapted to form a first fastening means. This minimises the surface area needed for the fastening means, and where mechanical and adhesive fastening means are used enables one region of adhesive to serve as both an adhesive fastening means (in one region) and an bonding region for a mechanical fastening means.

A region of material adapted to form a first fastening means may be provided on the first transverse side, and the region of material adapted to form a second fastening means, substantially co-extensive with and adapted to form a release surface for the first fastening means may be located on the second transverse side. This enables the hygiene article to be folded in half along its transverse centre line.

The at least one region of material adapted to form a second fastening means may be folded over onto the at least one region of material adapted to form a first fastening means.

The region of material adapted to form a second fastening means is preferably provided with a fingerlift portion extending beyond the region of material adapted to form a first fastening means. This enables each fastening means to be disengaged from the other easily by a user as there is a portion of the region of material adapted to form a second fastening means that can be held easily between fingers and thumb and so allowing the two fastening means to be peeled apart.

Preferably, the first fastening means is a mechanical fastening means, and the second fastening means is an adhesive fastening means. Alternatively, the first fastening means is an adhesive fastening means, and the second fastening means is a mechanical fastening means.

The material adapted to form the first fastening means and the material adapted to form the second fastening means may be the same type of material. The material is preferably a hermaphroditic mechanical fastening means or a releasable adhesive.

Preferably at least one mechanical fastening means comprises engaging projections formed from one of hooks, stems or cup-like projections.

The present invention will now be described by way of example and with reference to the accompanying drawings, in which:
Figure 1 is a diagrammatic top view showing a hygiene article with wings in accordance with a first embodiment of the present invention;
Figure 2 is a diagrammatic enlarged cross-section of a hygiene article in accordance with the first embodiment of the present invention illustrating adhesive fastening means and mechanical fastening means positioned for the hygiene article to be stored;
Figure 3 is a diagrammatic enlarged cross-section of a hygiene article in accordance with the first embodiment of the present invention illustrating the adhesive fastening means and the mechanical fastening means positioned ready for the hygiene article to be used by a user;
Figure 4 is a diagrammatic enlarged cross-section of a hygiene article in accordance with the first embodiment of the present invention in use, illustrating the adhesive fastening means and the mechanical fastening means in contact with a user's undergarment;
Figure 5 is a diagrammatic enlarged cross-section illustrating the method of attaching a mechanical fastening means to a hygiene article in accordance with the first embodiment of the present invention;
Figure 6 is a diagrammatic bottom view of a hygiene article in accordance with a second embodiment of the present invention;
Figure 7 is a diagrammatic bottom view of a hygiene article in accordance with a third embodiment of the present invention; and
Figure 8 is a diagrammatic side view of a hygiene article in accordance with the third embodiment of the present invention in a folded position.

The present invention adopts the approach that two different fastening means can be used to affix a hygiene article to the undergarment or other clothing of a wearer, and that each fastening means can be used as a protective cover to protect the other fastening means before the hygiene article is used. For example, if a mechanical fastening means and an adhesive fastening means are used, the mechanical fastening means acts as a release surface for the adhesive fastening means, and the adhesive fastening means covers the whole of the mechanical fastening means. To enable this, each fastening means is substantially co-extensive with the other fastening means, that is, each mechanical fastening means covers substantially the whole the adhesive fastening means to which it corresponds. In a first embodiment, the present invention is particularly suitable for use with hygiene articles such as sanitary napkins or towels, or adult incontinence products (each of which could employ wings for securing the hygiene article and preventing leakage of fluids or other bodily exudates). In a second embodiment, the present invention is particularly suitable for use with hygiene articles such as adult incontinence pads or sanitary napkins that do not employ wings, for example, panty liners. In a third embodiment, the present invention is also suitable for use with hygiene articles such as adult incontinence pads or sanitary napkins that do not employ wings, for example, panty liners.

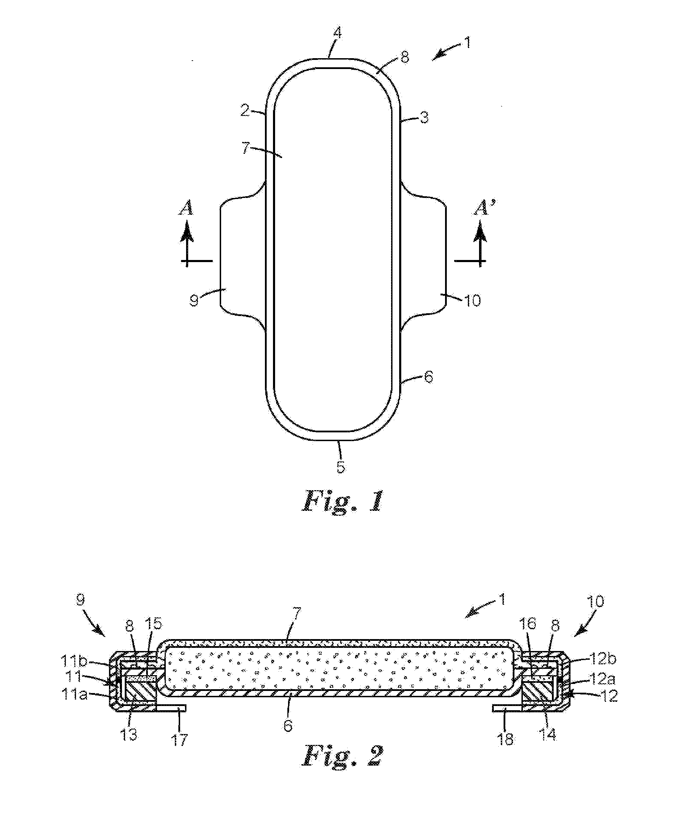

Figure 1 is a diagrammatic top view showing a hygiene article with wings in accordance with a first embodiment of the present invention. The hygiene article 1 is generally elongate in shape, having opposing first 2 and second 3 longitudinal sides (that is, the long sides of a substantially rectangular elongate shape) and opposing first 4 and second 5 opposing transverse sides (that is, the short sides of a substantially rectangular elongate shape). It is formed from an absorbent core sandwiched between a back sheet 6 and a front sheet 7, which both extend beyond the absorbent core sufficiently to form a sealing edge 8. The hygiene article 1 is provided with first 9 and second 10 wings, sometimes known as side flaps, provided on each of the first 2 and second 3 longitudinal sides, approximately half way along their length. The wings 9, 10 are preferably shaped to form rectangular portions lying with the long side of the rectangle along the sealing edge 8, although many other shapes, such as trapezoidal or semi-circular are also suitable. The absorbent core is preferably formed from a compressible, conformable material capable of absorbing and/or retaining liquids and other bodily exudates. Suitable materials include comminuted wood pulp (airfelt) creped cellulose wadding, absorbent foams, absorbent sponges, superabsorbent polymers, absorbent gelling materials or a combination of any of these. The absorbent core may be substantially rectangular in shape, or may be shaped to follow the outline of the hygiene article 1, that is, comprise cut-out regions corresponding to the leg position of a wearer. The back sheet 6 may be made from a flexible liquid impermeable material such as a thin plastic film, for example, polyethylene film, having a thickness in the range of 0.01mm to 0.05mm. The top sheet 7 may be formed from a liquid permeable material such as a porous foam, a porous film, or a woven or non-woven fabric of natural (for example wood or cotton) and/or synthetic (for example polyester or polyethylene) fibres. If desired, the top sheet 7 may also have hydrophobic properties to prevent the wearers' skin from coming into contact with liquids or other bodily exudates. The sealing edge 8 may be formed by bonding the back sheet 6 and the front sheet 7 together using an adhesive or other bonding process, such as thermal bonding or ultrasonic welding. The sealing edge 8 ext ends beyond the absorbent core sufficiently to ensure that the absorbent core is held firmly in position and is covered completely by the back sheet 6 and the front sheet 7. If desired, the absorbent core can be bonded to the back sheet 6 using an adhesive, or can be held in position between the back sheet 6 and the front sheet 7 purely by the presence of the sealing edge 8.

The first 9 and second 10 wings are preferably discrete and formed separately to the back sheet 6 and front sheet 7, and comprise a conformable non-woven tape material provided with a layer of a pressure sensitive adhesive material (not shown), a portion of which acts as an adhesive fastening means for the hygiene article 1. The non-woven tape material is bonded onto the top sheet 7 at the sealing edge 8 using an adhesive. Alternative bonding methods may be used if desired, such as ultrasonic or thermal welding. Suitable non-woven tape materials include Micropore™ and Transpore™ materials available from 3M Company, 3M Center, St. Paul, MN 55144-1000, USA. These materials are suitable as they offer a thin, conformable material with a medium tack that enables easy release from hook-type mechanical fastening means, as described below.

Figure 2 is a diagrammatic enlarged cross-section of a hygiene article in accordance with the first embodiment of the present invention illustrating adhesive fastening means and mechanical fastening means positioned for the hygiene article to be stored. This view is taken along the line A-A' shown in Figure 1, but enlarged for clarity. The first 9 and second 10 wings are each provided with a layer of pressure sensitive adhesive 11, 12 on the underside of the non-woven tape material layer. Each layer of pressure sensitive adhesive 11, 12 is divided into two regions to create first 11a and second 12a adhesive fastening means and first 11b and second 12b wing fixation means. These layers of pressure sensitive adhesive 11, 12 are therefore regions of material adapted to form a first fastening means11a, 12a. The first 11b and second 12b wing fixation means are used to adhere the wings 9, 10 to the sealing edge 8 of the hygiene article 1. However, first 11b and second 12b wing fixation means may not be present if other ways of bonding the wings 9, 10 to the sealing edge 8 are used, for example, ultrasonic welding, thermal bonding or any combination of known bonding means with or without an adhesive bonding means. The first 11a and second 12a adhesive fastening means are used to adhere the hygiene article 1 to the clothing of a wearer. Regions of a second material are adapted to form a second fastening means in the form of first 13 and second 14 mechanical fastening means. These first 13 and second 14 mechanical fastening means are provided on the first 2 and the second 3 longitudinal sides in the region of the wings 9, 10. Each is arranged on the underside of the sealing edge 8 and bonded to the back sheet 6 of the hygiene article 1 by a layer of adhesive 15, 16. The mechanical fastening means 13, 14 are substantially the same width as the sealing edge 8, and extend along the longitudinal sides 2, 3 of the hygiene article 1 for substantially the same distance as the length of the wings 9, 10. Preferably, the mechanical fastening means 13, 14 comprise a hook-type mechanism, which includes fasteners having hook-shaped projections (which may be "J"- or "T"-shaped), stem-like projections (that rely solely on friction with the threads of a garment) or cup-like projections, such as those described in WO01/10373, to which reference should be made.

Each of the mechanical fastening means 13, 14 acts as a release surface for the adhesive fastening means 11a, 12a provided on the wings 9, 10. The adhesive fastening means 11a, 12a are substantially co-extensive with the mechanical fastening means 13, 14, that is, each adhesive fastening means 11a, 12a covers the substantially the whole of the mechanical fastening means 13, 14 to which it corresponds. In this manner, the adhesive fastening means 11a, 12a are adapted to form a protective cover for the mechanical fastening means 13, 14, in particular, when the hygiene article 1 is stored and/or before the hygiene article 1 is used. In order to enable the mechanical fastening means 13, 14 to act as a true release surface for the adhesive fastening means 11a, 12a, the bond between the pressure sensitive adhesive material and the hook mechanism of the mechanical fastening means 13, 14 must be broken easily by a user without damage to either the adhesive 11a, 12a or the mechanical fastening means 13, 14. This is achieved firstly by the reduced surface area of the hooks forming the hook mechanism providing a small area with which a non-permanent adhesive bond can be made by the pressure sensitive adhesive material, and secondly also by providing a fingerlift portion 17, 18 at the end of each of the adhesive fastening means 11a, 12a. An additional release coating may also be provided on the hooks of the hook mechanism. This fingerlift portion 17, 18 is formed from an adhesive-free region of the non-woven tape material forming the wings 9, 10, and is sized so as to be easily grasped by a user as it extends beyond the mechanical fastening means 13, 14. A separate fingerlift portion 17, 18 that is attached to the non-woven tape material forming the wings 9, 10 may be used as an alternative, if desired. Fingerlift portions are disclosed in US 3, 895, 460 and US 4, 553, 560, by way of example.

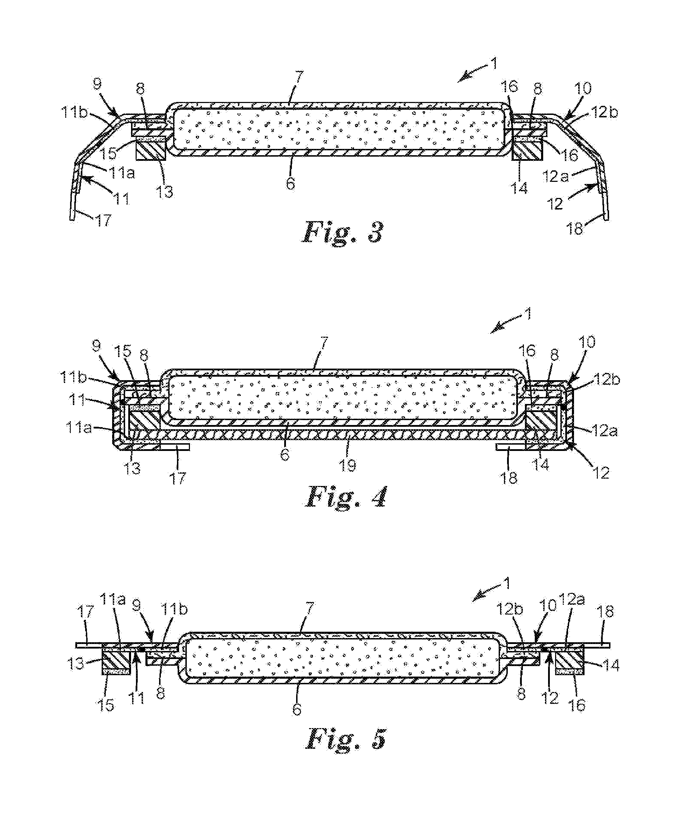

Figure 3 is a diagrammatic enlarged cross-section of a hygiene article in accordance with the first embodiment of the present invention illustrating the adhesive fastening means and the mechanical fastening means positioned ready for the hygiene article to be used by a user. Again, this view is taken along the line A-A' shown in Figure 1, but enlarged for clarity. In order to use the hygiene article 1, a user must remove the adhesive fastening means 11a, 12a from the mechanical fastening means 13, 14, so that the mechanical fastening means 13, 14 are exposed. This is done by lifting the finger lift portions 17, 18 and gently pulling the adhesive fastening means 11a, 12a away from the release surface formed from the mechanical fastening means 13, 14. This allows the wings 9, 10 to be opened outwards, as shown in Figure 3, so that the hygiene article 1 can be placed easily against the inside of a wearer's undergarment. By opening out the wings 9, 10 the mechanical fastening means 13, 14 are exposed, and when the hygiene article 1 is placed in contact with a wearer's undergarment the hook mechanism of the mechanical fastening means 13, 14 engages with the fibres of the undergarment, penetrating the weave of the fibres to create a friction fit. When the hygiene article 1 is worn in use, the wings 9, 10 act to prevent leakage of fluids or other bodily exudates from the main body of the hygiene article 1, and protect the edges of the undergarment from soiling. They also help to maintain the position of the hygiene article 1 as the wearer moves around.

This situation is shown in Figure 4. Figure 4 is a diagrammatic enlarged cross-section of a hygiene article in accordance with the first embodiment of the present invention in use, illustrating the adhesive fastening means and the mechanical fastening means in contact with a user's undergarment. Again, this view is taken along the line A-A' shown in Figure 1, but enlarged for clarity. Once the hygiene article 1 has been placed against the inner side of a wearer's undergarment 19 and the mechanical fastening means 13, 14 have engaged with the fibres of the undergarment 19, as described above, the wings 9, 10 are folded around the edges of the undergarment 19 and the adhesive fastening means 11a, 12a adhered to the fibres of the outer side of the undergarment 19. In this position, the wings 9, 10 act to prevent leakage of fluids or other bodily exudates from the main body of the hygiene article 1, and protect the edges of the undergarment from soiling. They also help to maintain the position of the hygiene article 1 as the wearer moves around.

In addition to the mechanical fastening means 13, 14 provided in the region of the wings 9, 10, and the adhesive fastening means 11a, 12a provided on the wings 9, 10, other fastening means may be provided on the back sheet 6 of the hygiene article 1. For example, a strip of pressure sensitive adhesive could be provided centrally on the hygiene article 1 along its length, or regions/strips of pressure sensitive adhesive could be provided on the front and rear portions of the hygiene article in positions representing the optimum position for ensuring the hygiene article 1 remains in its intended position. As an alternative, strips or regions of mechanical fastening means could be used in the same manner. Each of these additional fastening means may be covered by a release liner or other suitable protective covering for storage, if required.

Although in the above description the wings 9, 10 are provided separately from the back sheet 6 and front sheet 7 of the hygiene article 1, as an alternative construction they may be formed from extensions of the back sheet 6 and/or the front sheet 7 extending beyond the sealing edge 8 and are preferably shaped to form rectangular portions lying with the long side of the rectangle along the sealing edge 8, although many other shapes are suitable, including trapezoidal and semi-circular. In this situation, the adhesive fastening means 11a, 12a may be provided by an adhesive coating or adhesive strips, positioned on the surface of each wing 9, 10 that contacts the outside of a wearers' undergarment. In addition, rather than providing a single adhesive fastening means 11a, 12a on each wing and a single mechanical fastening means 13, 14 on each sealing edge in the region of the wing, several of each type may be provided, for example, two, three or four discrete regions or strips. The fastening means may be provided symmetrically about an axis disposed longitudinally along the centre of the hygiene article or otherwise.

A further alternative is to change the positions of the adhesive 11a, 12a and mechanical 13, 14 fastening means with respect to each other. For example, mechanical fastening means 13, 14 could be positioned on the wings 9, 10, and adhesive fastening means 11a, 12a could be positioned on the back sheet 6 on the sealing edge 8. In this situation, the mechanical fastening means 13, 14 would be sized so as to be co-extensive with the adhesive fastening means 11a, 12a, and adapted to protect the adhesive fastening means 11a, 12a. The mechanical fastening means 13, 14 would also act as a release surface for the adhesive fastening means 11a, 12a in the same manner as described above. A further option is to provide alternating regions of first and second fastening means in a single position on the hygiene article. This may be achieved, for example, by replacing the adhesive fastening means 11a, 12a, on the wings 9, 10 each of which is formed as a single region with a region divided into two parts: one of which is a first fastening means, for example, an adhesive fastening means11a, 12a, and one of which is a second fastening means, for example a mechanical fastening means 13, 14. In order for the adhesive fastening means 11a, 12a provided on the wing 9, 10 to form a cover for the mechanical fastening means 13, 14 provided on the sealing edge 8, the positions of the adhesive fastening means 11a, 12a and the mechanical fastening means 13, 14 on the sealing edge 8 are reverse with respect to those on the wing9, 10. This means that each adhesive fastening means 11a, 12a corresponds to a mechanical fastening means 13, 14 and vice versa. A single adhesive fastening 1a, 12a means may protect one or more mechanical fastening means 13, 14, or a single mechanical fastening means 13, 14 may act as a release surface for one or more adhesive fastening means 11a, 12a.

Figure 5 is a diagrammatic enlarged cross-section illustrating the method of attaching a mechanical fastening means to a hygiene article in accordance with the first embodiment of the present invention. In order to affix the mechanical fastening means 13, 14 to the sealing edge 8, initially the wings 9, 10 are provided with the mechanical fastening means 13, 14 positioned face down on the layer of pressure sensitive adhesive material 11, 12 provided on the underside of the non-woven tape material, that is with the hook-type mechanism in contact with the layer of adhesive material in the position that will eventually form the first 11a and second 12a adhesive fastening means. The base of the mechanical fastening means 13, 14 is provided with adhesive 15, 16 to bond the mechanical fastening means 13, 14 to the back sheet 6 on the sealing edge 8. To create this bond, the wings 9, 10 are folded over such that the adhesive 15, 16 on the base of the mechanical fastening means 13, 14 contacts and forms an adhesive join with the back sheet 6. The layer of adhesive 15, 16 bonding the mechanical fastening means 13, 14 to the back sheet 6 has a greater adhesive strength to both the back sheet 6 and the base of the mechanical fastening means 13, 14 than the adhesive strength of the pressure sensitive adhesive forming the adhesive fastening means 11a, 12a to the hook-type mechanism of the mechanical fastening means 13, 14. This ensures that when the adhesive fastening means 11a, 12a are removed from the mechanical fastening means 13, 14, the mechanical fastening means 13, 14 act as a release surface and remain firmly bonded to the back sheet 6.

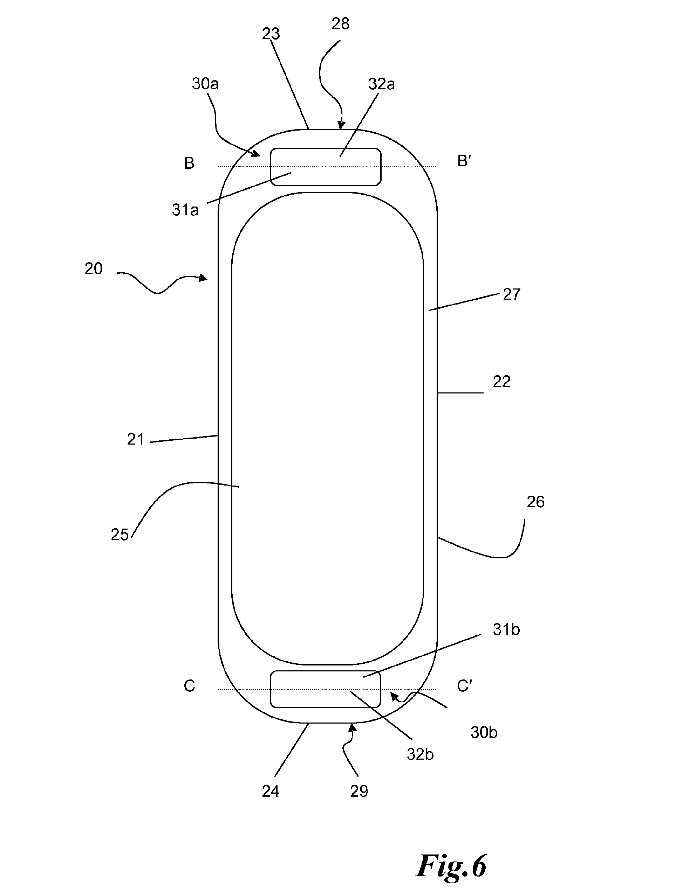

Figure 6 is a diagrammatic bottom view of a hygiene article in accordance with a second embodiment of the present invention. In this embodiment, a hygiene article 20 is generally elongate, having first 21 and second 22 longitudinal sides and first 23 and second 24 transverse sides. Again, the hygiene article 20 is formed from an absorbent core sandwiched between a back sheet 25 and a front sheet 26, which both extend beyond the absorbent core sufficiently to form a sealing edge 27. At the first 23 and second 24 transverse sides the back sheet 25 and the front sheet 26 extend further from the absorbent core than along the first 21 and second 22 longitudinal sides, forming first 28 and second 29 sealing edge extensions. For a hygiene article 20 such as an adult incontinence pad, which is typically 25 cm in width and 40 cm in length, these sealing edge extensions 28, 29 may have an extension beyond the absorbent core by between 1 cm and 3 cm.

In Figure 6, the back sheet 25 is shown as being uppermost. A fixation area 30a, 30b is provided on the sealing edge extensions 27, 28, each of which comprises a region of material adapted to form a first fastening means in the form of an adhesive fastening means 31a, 31b and a region of material adapted to form a second fastening means in the form of a mechanical fastening means 32a, 32b, placed adjacent to each other. This means that each of the first and second fastening means are provided on the first 23 and the second 24 transverse sides. As in the first embodiment of the present invention described above, the mechanical fastening means 32a, 32b comprise a hook-type mechanism, and the adhesive fastening means 31a, 31b comprise a pressure sensitive adhesive material. The adhesive fastening means 31a, 31b are positioned directly on the back sheet 25 of the hygiene article 20, in the form of an adhesive coating rather than being provided as a layer of adhesive on the reverse of a non-woven tape material. Other types of pressure sensitive adhesive, such as tapes, are also suitable. A region of adhesive having a surface area in the range of approximately 5-20 cm² is provided on the fixation areas 30a, 30b to form both the adhesive fastening means 31a, 31b and an bonding region for the mechanical fastening means 32a, 32b. The mechanical fastening means 32a, 32b are bonded to the back sheet 25 by this region of adhesive, and cover approximately 50% of the surface area of the adhesive region. The region of adhesive is positioned such that a portion of the sealing edge 27 may be used as a fingerlift portion to provide easy removal of the mechanical fastening means 32a, 32b from the adhesive fastening means 31a, 31b. Both the mechanical fastening means 32a, 32b and the mechanical fastening means 31a, 31b are provided in the form of an oblong-shaped region, located symmetrically about an axis positioned centrally between the first 21 and the second 22 longitudinal sides (the longitudinal centre line).

The mechanical fastening means 32a, 32b are adapted to protect the adhesive fastening means 31a, 31b, as when the sealing edge extensions 28, 29 are folded along the fold lines B-B' and C-C' in Figure 6 (both of which run along the boundary between the adhesive fastening means 31a, 31b, and the mechanical fastening means 32a, 32b) the mechanical fastening means 32a, 32b cover the adhesive fastening means 31a, 31b. The mechanical fastening means 32a, 32b, cover approximately 50% of the region of adhesive leaving approximately 50% free to form the adhesive fastening means 31a, 31b, such that the mechanical fastening means 32a, 32b are co-extensive with the adhesive fastening means 31a, 31b when the article is in the storage position. To store the hygiene article 20, the sealing edge extensions 28, 29 are folded along the fold lines B-B' and C-C, such that the mechanical fastening means 32a, 32b cover the adhesive fastening means 31a, 31b and act as a release surface for the adhesive fastening means 31a, 31b, in the same manner as described above with respect to the first embodiment of the present invention. The advantage in providing both adhesive fastening means 31a, 31b and mechanical fastening means 32a, 32b on the sealing edge extensions 28, 29 is that both sit within the original footprint of the hygiene article 20, and additional side or end flaps are not required.

In addition to the adhesive fastening means 31a, 31b and mechanical fastening means 32a, 32b provided on the sealing edge, other fastening means may be provided elsewhere on the back sheet 25 of the hygiene article 20, for example, a strip of pressure sensitive adhesive material covered with a release liner positioned on a central portion of the hygiene article 20. Also, rather than providing a single adhesive 31a, 31b and a single mechanical 32a, 32b fastening means on each sealing edge extension 28. 29, it may be desirable to provide several, for example, two, three or four, of each fastening means adjacent to each other. A further alternative is to position both the mechanical fastening means 32a, 32b and the adhesive fastening means 31a, 31b on the back sheet 25 such that one type of fastening means is located in the region of the absorbent core, such that the fold line between the two types of fastening means is located along the periphery of the absorbent core. In this situation, only one type of fastening means need be provided on the sealing edge. This removes the need to provide additional material in the form of the sealing edge extensions 28, 29, and any consequential re-sizing of the hygiene article and/or its component materials.

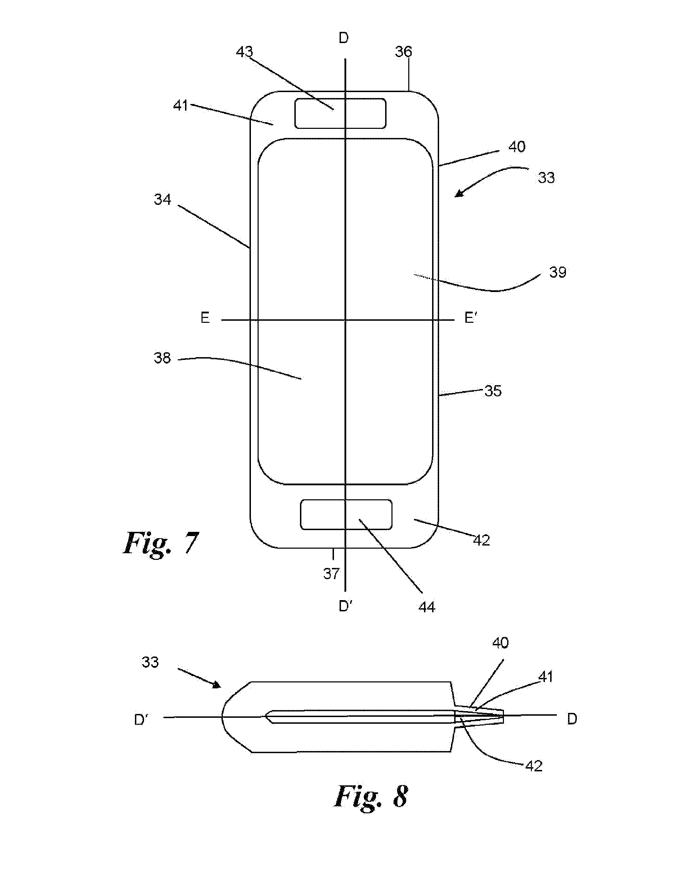

Figure 7 is a diagrammatic bottom view of a hygiene article in accordance with a third embodiment of the present invention. This shows the back sheet of the hygiene article in the uppermost position, as in Figure 6 above. This embodiment of the present invention is suitable for use with both sanitary napkins and adult incontinence pads. In this embodiment, a hygiene article 33 is generally elongate, having first 34 and second 35 longitudinal sides and first 36 and second 37 transverse sides. Again, the hygiene article 33 is formed from an absorbent core sandwiched between a back sheet 38 and a front sheet 39, which both extend beyond the absorbent core sufficiently to form a sealing edge 40. At the first 36 and second 37 transverse sides the back sheet 38 and the front sheet 39 extend further from the absorbent core than along the first 34 and second 35 longitudinal sides, forming first 41 and second 42 sealing edge extensions. An adhesive fastening means 43 is located on the back sheet adjacent the sealing edge 40 at the first transverse side 36, on the first sealing edge extension 41, and a mechanical fastening means 44 is located on the back sheet adjacent the sealing edge at the second transverse side 37, on the second sealing edge extension. Both the adhesive fastening means 43 and the mechanical fastening means 44 are provided in the form of an oblong-shaped region, located symmetrically about the axis D-D' (the longitudinal centre line) positioned centrally between the first 34 and the second 35 longitudinal sides. Each of these oblong-shaped regions extends parallel to the first 36 and second 37 transverse sides, and covers approximately 60% of the width of the hygiene article 33. Again, preferably the mechanical fastening means 44 comprises a hook-type mechanism, and the adhesive fastening means 43 comprises a pressure sensitive adhesive material.

By providing the adhesive fastening means 43 and the mechanical fastening means 44 at opposite transverse sides 36, 37 of the hygiene article 33, the hygiene article 33 may be folded about an axis E-E' (the transverse centre line) positioned halfway between and parallel to the first and second transverse sides 36, 37. When folded, the adhesive fastening means 43 and the mechanical fastening means 44 come into contact with one another, such that the mechanical fastening 44 means is adapted to form a protective cover for the adhesive fastening means 43. This is achieved by ensuring that the mechanical fastening means 44 is substantially co-extensive with the adhesive fastening means 43.

Figure 8 is a diagrammatic side view of a hygiene article in accordance with the third embodiment of the present invention in a folded position. This illustrates how the hygiene article 33 is folded along E-E' such that the adhesive fastening means 43 and the mechanical fastening means 44 are brought into contact with one another. The hygiene article 33 is maintained in the folded position by means of the adhesion between the adhesive fastening means 43 and the mechanical fastening means 44, which is overcome by peeling apart the first 36 and second 37 transverse sides of the hygiene article 33. As described above, the mechanical fastening means 44 form a release surface for the adhesive fastening means 43, but in this case, the choice of adhesive and the choice of mechanical fastening means is also determined not only by how easily one may be released from the other, but by considering the requirement that there should be sufficient adhesion between the two types of fastening means to ensure that the hygiene article 33 remains in a folded position. For example, the peel force required to separate the mechanical fastening means 44 and the adhesive fastening means 43 should be sufficient to resist any force applied as a result of any compression or tension within the component materials of the hygiene article 33 in the folded position. However, depending on the position of the fastening means it may be desirable to fold the hygiene article 33 along the longitudinal axis D-D' as an alternative. This would involve splitting the adhesive fastening means 43 and the mechanical fastening means 44 about the longitudinal axis D-D', rather than the transverse axis E-E'.

As an alternative, both the adhesive fastening means 43 and the mechanical fastening means 44 may be positioned anywhere on the back sheet 38 as desired. For example, each could be positioned in the region of the sealing edge 40 that is proximate or adjacent to the sealing edge 40, or away from the sealing edge 40 on the main body of the hygiene article 33.

In each of the embodiments described above, regions of material adapted to form the fastening means comprise two distinct types of fastening means: a mechanical fastening means and an adhesive fastening means. However, this does not need to be the case. Each of the regions of material adapted to form the fastening means may in fact be the same type of material. For example, fastening means known as hermaphroditic fastening means, where rather than using a hook and loop fastening system (where hooks engage or otherwise mesh with opposing loops or fibrous material) each half of the fastening system is the same. This may be achieved by using a mechanical fastening means comprising projections, having a stem extending from a base, and a head portion overhanging the stem. When a region of such projections is placed into contact with a second region of the same projections and pushed together, the projections mate together as the heads of one region of projections penetrate the areas between the stems of the other region of projections, causing the regions to mesh together. By forming the projections from a flexible and/or resilient material, the projections may be meshed together, un-meshed and re-meshed many times. This makes such materials ideal for use in the present invention, where one region of projections may form a first fastening means, and a second region of projections may form a second fastening means, adapted to form a protective cover for the first fastening means, and sized so as to be substantially co-extensive with the first fastening means.

A second example of materials that have such hermaphroditic properties are a particular class of adhesives, such as those disclosed in US 4,513,039. These adhesives are releasable adhesives, in that they may be placed into contact with each other but peeled apart without damage. This means that a first region of adhesive can form a first fastening means, and a second region of the same adhesive can form a second fastening means, adapted to form a protective cover for the first fastening means, and sized so as to be substantially co-extensive with the first fastening means. Hence one region of adhesive is able to act as a release surface for the other region of adhesive and vice versa. Furthermore, combinations of mechanical fastening means and adhesives are known, for example, the fastening system disclosed in US 4,959,265, where an adhesive is provided at the base of the stems of the mechanical fastening means. In each of these cases the fastening means may be of exactly the same material, or two different materials having identical or similar properties.

## Claims

1. A hygiene article, comprising:
an absorbent core having an elongate shape sandwiched between a back sheet and a front sheet, the back sheet and the front sheet extending beyond the absorbent core and being sealed together to form a sealing edge portion;
a region of material adapted to form a first fastening means arranged on the back sheet; and
a region of material adapted to form a second fastening means, substantially co-extensive with, and adapted to form a protective cover for, the first fastening means.

2. A hygiene article according to claim 1, wherein the region of material adapted to form a first fastening means is arranged on the back sheet in the region of the sealing edge.

3. A hygiene article according to claim 1, wherein the region of material adapted to form a first fastening means is arranged on the back sheet on the sealing edge.

4. A hygiene article according to claim 1, 2 or 3, wherein the region of material adapted to form a second fastening means is arranged on the back sheet on the sealing edge.

5. A hygiene article according to claim 1, 2 or 3, further comprising a first wing having at least one region of material adapted to form a second fastening means located thereon and a second wing having at least one region of material adapted to form a second fastening means located thereon.

6. A hygiene article according to claim 1, 2 or 3, wherein the article comprises first and second opposing longitudinal sides and first and second opposing transverse sides, with at least one region of material adapted to form a first fastening means provided on the first longitudinal side and at least one region of material adapted to form a first fastening means provided on the second longitudinal side.

7. A hygiene article according to claim 5, wherein the wings are formed from the front sheet and/or the back sheet.

8. A hygiene article according to claim 5, wherein the wings are discrete and bonded to the hygiene article at the sealing edge.

9. A hygiene article according to claim 1, 2 or 3, wherein the article comprises first and second opposing longitudinal sides and first and second opposing transverse sides, with at least one region of material adapted to form a first fastening means provided on the first transverse side and at least one region of a material adapted to form a first fastening means provided on the second transverse side.

10. A hygiene article according to claim 9, wherein a region of material adapted to form a second fastening means is provided adjacent to each region of material adapted to form a first fastening means.

11. A hygiene article according claim 9, wherein a region of material adapted to form a first fastening means is provided on the first transverse side, and the region of material adapted to form a second fastening means, substantially co-extensive with and adapted to form a release surface for the first fastening means is located on the second transverse side.

12. A hygiene article according to any preceding claim, wherein the at least one region of material adapted to form a second fastening means is folded over onto the at least one region of material adapted to form a first fastening means.

13. A hygiene article according to any preceding claim, wherein the second fastening means is provided with a fingerlift portion extending beyond the at least one first fastening means.

14. A hygiene article according to any preceding claim, wherein the first fastening means is a mechanical fastening means, and the second fastening means is an adhesive fastening means.

15. A hygiene article according to any of claims 1 to 13, wherein the first fastening means is an adhesive fastening means, and the second fastening means is a mechanical fastening means.

16. A hygiene article according to any of claims 1 to 13, wherein the material adapted to form the first fastening means and the material adapted to form the second fastening means are the same type of material.

17. A hygiene article according to claim 16, wherein the material is a hermaphroditic mechanical fastening means or a releasable adhesive.

18. A hygiene article according to any of claims 14 to 17, wherein the at least one mechanical fastening means comprises engaging projections formed from one of hooks, stems or cup-like projections.
